# EUROPEAN PATENT APPLICATION

(11) **EP 2 749 271 A1**
(43) Date of publication of application: **02.07.2014**
(21) Application number: 12199818.1
(22) Date of filing: 31.12.2012
(51) Int. Cl.: A61K 9/20, A61K 31/506

(54) **Optimized manufacturing method and pharmaceutical formulation of imatinib**

(71) Applicant: Deva Holding Anonim Sirketi, 34303 Istanbul (TR)
(72) Inventor: Koc, Fikret, 34303 Istanbul (TR); Kandemir, Levent, 34303 Istanbul (TR); Firat, Omer Faruk, 34303 Istanbul (TR); Ilhan, Suna Ayse, 34303 Istanbul (TR)

(57) **Abstract**

This invention relates to an optimised manufacturing method and a stable pharmaceutical formulation for high load of imatinib or pharmaceutical salt thereof for use in solid oral compositions.

## Description

### Technical Field

This invention relates to an optimised manufacturing method and a stable pharmaceutical formulation for high load of imatinib or pharmaceutical salt thereof for use in solid oral compositions.

### Background Art

Imatinib is a synthetic tyrosine kinease inhibitor which is the first member of a new class of agents which acts by inhibiting a certain enzyme that is characteristic of a particular cancer cell.

Imatinib is used to treat chronic myelogenous leukemia (CML) and gastrointestinal stromal tumors (GISTs) and can be administered to patients in daily doses ranging from 100 mg to 800 mg depending on the condition of patient. Once daily administered 400 mg of imatinib is the standard treatment of Philadelphia positive (Ph+) chronic myeloid leukemia( CML).

EP 0564409 B (NOVARTIS AG) 19.01.2000 firstly discloses imatinib free base and processes for its manufacture. But, imatinib free base is practically insoluble in water and cannot be considered for development. As it displays good solid state properties and stability, methane sulphonic acid salt is preferred. Imatinib mesylate, is chemically 4-[(4-Methyl-1-piperazinyl) methyl]-N-[4-methyl-3-[[4-(3-pyridinyl)-2-pyrimidinyl] amino] phenyl]-benzamide mesylate.

Imatinib mesylate and its two crystalline forms; alpha and beta crystal forms are disclosed in WO 99/03854 A (NOVARTIS-ERFINDUNGEN VERWALTUNGSGESELLSCHAFT MBH) 28.01.1999

The alpha form of imatinib mesylate is characterized by needle shape crystals which have unfavourable flow properties. Besides, the alpha crystalline of imatinib mesylate is also known to be thermodynamically unstable at elevated temperature which means that the process temperature should be maintained controlled.

### Summary of invention

This invention relates to an optimised manufacturing process and a stable pharmaceutical formulation for imatinib or pharmaceutical salt thereof for use in solid oral compositions.

### Technical Problem

Depending on the characteristics of active ingredient and the required final granules, an optimised manufacturing method and a suitable pharmaceutical formulation are needed to be developed by formulation scientist in drug product development.

In the case of imatinib tablet manufacture, development of a robust tablet formulation of imatinib is challenging for formulation scientist. Two main problems arise when different industrial manufacturing equipments are used.

As prescribed daily dosage of imatinib for treatment of leukemia is high, a convenient, patient friendly, an appropriately sized solid oral dosage formulation of imatinib is required to be developed.

The core imatinib tablet contains 400 mg of imatinib free base (equivalent to 478 mg of imatinib mesylate salt) and other additional pharmaceutical excipients in order to fulfill the requirements with respect to technical properties.

Low density's pharmaceutical excipients which have the functions of guarantying the dosage, stability and bioavailability, constitute the greater volume of imatinib tablet.

Thus, weights of excipients which add to weight of active ingredient increase greatly the weight of total tablet wherein bulk density of mixture to be filled is decreased.

The low bulk density of powder mixture negatively impacts processing operations, causes that the required quantity of powder physically will not fit into the die cavity on the tablet press. Fluffy drug excipient blend usually results in excessive tablet weight variations in manufacturing.

On the other hand, the needle crystalline shape of imatinib impacts flow properties of drug-excipient blend in manufacturing process and complicates the manufacturability of solid oral dosage form of imatinib.

The appropriate manufacturing process technique of imatinib should be designed and selected to circumvent any potential polymorphic transformations. The conversion of alpha crystalline form of imatinib into beta form is especially undesirable and avoided.

Raw material cohesiveness of imatinib is another issue to be considered when developing high load solid dosage form of imatinib.

During studies, inventors realised that densification and agglomeration of imatinib granules have increased if wet massing has not been completed quickly. Due to high solubility of active ingredient, imatinib mesylate becomes cohesive and sticky in contact with water during wet massing step. Granules are hardening very quickly before granulation's end point.

While it is often fundamental to develop the best possible pharmaceutical formulation, in practice, selection of the correct manufacturing technique and/ or equipment is equally important in the development of pharmaceutical composition comprising imatinib or pharmaceutically acceptable salts thereof. Consequently, a well defined granulation process is particularly indispensable for hardly processable imatinib to ensure reproducible granule quality thus tablet properties.

Formulations, processes are specially needed to be developed with regards to problem arising to improve quality of granules and finished products containing imatinib or pharmaceutically acceptable salt thereof.

### Solution to Problem

The inventors aimed to develop a pharmaceutical formulation which contains a high content of imatinib, with a relatively small amount of excipients to enable the production of physically small tablets. In this respect, different manufacturing methods and pharmaceutical formulations are evaluated in by our inventors in order to achieve suitable sized tablet for high load of imatinib tablet.

Our inventors have surprisingly found that imatinib must be densified in manufacturing of final solid dosage form so that sufficiently high doses can be administered in a reasonably sized dosage form. In this respect, densification of active ingredient is aimed in order to facilitate dosing of the active ingredient.

It is an object of the present invention to provide a process for a solid pharmaceutical composition comprising imatinib or pharmaceutical salts thereof wherein a partial amount of active ingredient is added intragranularly to granulator to produce high load imatinib tablet. The remaining part of active ingredient is added extragranularly.

In the second aspect of the present invention, as it provides short granulation time, high density and high strength granules; inventors optimized the high shear granulation for high load formulation of imatinib.

The optimised granulation process is developed to achieve easy, economical process to obtain stable, consistent, high quality granular appropriate tablet properties and process control.

### Description of embodiments

Inventors are undertaken investigations on both developments of different pharmaceutical formulations and manufacturing methods (including wet and dry granulation and direct compression) for high load dose of solid oral formulations of imatinib.

Preliminary investigations of our inventors have resulted that direct compression method is not preferable for high dose imatinib tablet. Inventors found that encountered difficulties in the manufacturing imatinib or pharmaceutical acceptable salt thereof can be surpassed by the present invention.

Granulation consists of agglomeration of powder and is widely used for the purpose of improvement of physical and rheological properties of the powder of most pharmaceutical dosage forms. Wet granulation methods have been the most widely used powder granulation technology in production of pharmaceutical products.

Wet granulation in which components are typically mixed and granulated using a binder, is specially preferred to improve flow and compaction properties of the powder in the production of solid oral dosage forms.

The binder solution is added to the mechanically blended powder mix which results in particle size enlargement by formation of liquid bridges between primary particles.

A consistent and uniform granulation process and granules of optimal quality can be achieved when critical parameters are controlled. Therefore, the choices of process and apparatus are of great importance for final granule properties of imatinib.

In the present invention, final granules resulting from high shear, low shear and fluid bed granulations of high load of imatinib formulations are evaluated. Depending on both granulation process and design of pharmaceutical composition, imatinib granules with different characteristics are obtained. Final and critical parameters have been accordingly determined for the process.

The improvement in compression of final mixture and the reduction in bulk volume of voluminous powder of imatinib mixture are also assessed for selection of best results.

In the present invention, although all granulation methods may be used, inventors have been focused on a single pot mixer-granulator system which consists of one piece of equipment to carry out in the same equipment without discharging.

A single pot is a double jacket bowl apparatus equipped with an impeller and a chopper which allows mixing, granulating and drying in the same apparatus.

A mixer-granulator secures granulation of product, removes the need to transfer the product between pieces of equipment and so reduces the opportunity for segregation to occur. Used top driven high shear mixer granulator reaches each and every corner of the trough and imparts radial and linear motion to the whole of powder to be mixed.

The single-pot alone or in combination with the fluid-bed technology ensures granulation and drying processes with maximum efficiency.

It is known that wet massing time controls granule density of imatinib and using excessive levels of granulating fluids and binding agent give rise to sticking to internal wall of granulator which ends up in a low yielded production.

During studies, inventors realised that densification and agglomeration of imatinib granules have increased if wet massing haven't completed quickly.

Nevermore, formation of lump and non-homogeneous granules of granules are not disregarded in the present invention.

The inventors have scoped out the occurring problem and figured out by adjustments in process granulation and composition of formulation of high load imatinib.

The presented unit dosage form has imatinib or pharmaceutically acceptable salt and one or more pharmaceutically acceptable excipients in amounts sufficient to yield a pharmacokinetic profile. In leukaemia, the design of the pharmaceutical formulation of high daily dose of imatinib is compelling by reason of the size of tablet. This means that the formulation scientist has to do so that the tablet does not become too large.

In first aspect of this invention, an improved manufacturing method for a solid dosage form containing imatinib is disclosed.

The solid oral dosage forms comprising imatinib or a pharmaceutical acceptable salt wherein preparation has intra-granulation step and extra-granulation step. In another embodiment of the invention, the manufacturing method comprises an intragranular addition of a partial amount of imatinib.

In a second aspect of the present, an improved process of granulation for preparing a solid dosage form containing imatinib is disclosed.

Although all process parameters are critical for imatinib granules quality, speed of impeller, liquid addition rate, wet massing time influence primarily resulting granules.

The results shown in table 1 reveal that granules prepared by different granulating solvents and granulation techniques show different features.

For selection of best granulation conditions, the results are compared as follows.

**Table 1**

| Impeller Speed (rpm) | Total amount of Water (g) | Liquid Addition Rate (g/min) | Wet Massing Time (min) |
|---|---|---|---|
| 50 | 3500 | 700 | 5 |
| 50 | 3500 | 1400 | 2.5 |
| 100 | 3500 | 700 | 5 |
| 100 | 3500 | 1400 | 2.5 |
| 150 | 3500 | 1400 | 2.5 |
| 180 | 3500 | 1750 | 2.5 |

The above results clearly indicate that an optimized granulation occurs when impeller speed is 180 rpm with a liquid addition rate of 1750 g/min in 2.5 min of massing time.

The outlet air temperature is set to 80° C, the inlet air temperature is set to 35°C in the granulation process.

The designed process is proved to be less sensitive to operating conditions than tumbling granulators and produce small high density granules.

In third aspect of this invention, the resulting compositions which have desirable manufacturing characteristics, excellent mechanical properties, dissolution profiles are disclosed.

It is found that granules prepared in granulation technique show better properties at processibility, reproducibility, and powder flow features for high load oral solid dosage forms of imatinib.

On the other hand, granulate mass produced according to this invention is believed to have good compressibility.

The granules are analyzed for particle size distribution, bulk and tapped density and flow properties such as Hausner ratio, compressibility index.

The Hausner ratio may be calculated using formula pₜₐₚ/p_{bulk} where p ₜₐₚ represents tapped density of granulate mass and p_{bulk} represents the loose bulk density of granulate mass.

The Hausner ratio and compressibility index are both measures of the flow properties of powders. A Hausner ratio of <1.25 indicates a powder that is free flowing whereas >1.25 indicates poor flowability.

In a further aspect, granules with Hausner ratio of greater than 1, namely 1.24, 1.20 or 1.25 are obtained in the present invention.

Compressibility index is indirect mesure of the flowability of the powder and it is determined according to US Pharmacopeia General Chapter <1174>.

The smaller the compressibility index the better the flow properties. For example 5-15 indicates excellent, 12-16 good, 18-21 fair and >23 poor flow.

In a further aspect, the present invention relates to pharmaceutical or compositions including imatinib or pharmaceutically acceptable salt having compressibility index <20.

**Table 2**

| | **Tapped Density** **(g/ml)** | **Bulk Density** **(g/ml)** | **Hausner Ratio** **(pₜₐₚ/p_{bulk})** | **Compressibility Index** **(100*(1-p_{bulk}/pₜₐₚ))** |
|---|---|---|---|---|
| ¼ of intragranular imatinib | 0.471 | 0.538 | 1.14 | 12.50 |
| ½ of intragranular imatinib | 0.574 | 0.464 | 1.24 | 19.36 |
| ¾ of intragranular imatinib | Granulation cannot be completed | Granulation cannot be completed | | |

The above table clearly indicates the optimum partial amount of imatinib which should be added as intragranularly. The best result is obtained when ½ of total amount of imatinib is added intragranularly, wherein the rest of imatinib is added extragranularly to mixture.

In another aspect, the unit dosage form has imatinib or pharmaceutical acceptable salt and one or more pharmaceutically acceptable excipients as components. With a tablet dosage form the one or more excipients can be chosen from disintegrants, binders, diluents, glidants, lubricants, coloring agents, stabilizers, preservatives, and/or flavoring agents.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A@, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E@, F and K, Metolose SH® of Shin-Etsu, grades of Methocel F@ and Metolose 65 SH®, the 4,000, 15,000 and 100,000 cps grades of Methocel K@; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested .Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, micro fine cellulose, low-substituted hydroxypropylcellulose, mixtures thereof and the like.

Glidants improve the flowability of the composition. The composition may also comprise a glidant. Glidants are, but not limited to, colloidal silicon dioxide, calcium silicate, magnesium trisilicate, powdered cellulose, talc, tribasic calcium phosphate, mixtures thereof and the like.

The glidant is preferably present in 0.1 % to 5% by weight based on the total mass of the pharmaceutical composition, more preferably from 0.5% to 4%, most preferably from 0.5 % to 3%,

The presence of a lubricant is particularly preferred when the composition is a tablet as lubricants to improve the tableting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

The lubricant is preferably present in 0.1 % to 5% by weight based on the total mass of the pharmaceutical composition, more preferably from 0.5% to 4%, most preferably from 0.5% to 3%.

The following example is given for the purpose of illustration of the present invention and should not limit the scope of the invention.

Imatinib which is exemplified above may be used as either the free base or as one or more salts known to the art.

In another embodiment, the invention provides compositions and methods useful for preparing unit dosage forms having imatinib, or a pharmaceutically acceptable salt thereof, as the active pharmaceutical ingredient.

Although tablets and granules described are produced using a conventional tablets, additional processes can be used alternatively. For instance, a compactor can be used to break firstly compressed tablets. Following to compactor, broken tablets can be compressed again into tablets to increase density.

In the present invention, a single pot, high shear mixer-granulator meets the highest requirements. However, the formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods.
(a) charging a part of imatinib drug substance, microcrystalline cellulose, a part of crospovidone, hydroxypropyl methylcellulose in mixer-granulator;
(b) blending mixture;
(c) granulating the material using purified water;
(d) All the powders(drug and excipients)were brought to a standard particle size by passing through an appropriate size screen;
(e) drying the milled granulation;
(f) milling the dried granulation through an appropriate size screen;
(g) charging dried granulation along with microcrystalline cellulose, croscarmellose sodium, and colloidal silicon dioxide into a diffusion blender and blending the material for an appropriate amount of time;
(h) charging the magnesium stearate into the diffusion blender and blending for an appropriate amount of time;
(i) compressing the blended powders on a high-speed rotary press; and
(j) film coating the tablets.

The core tablets and core granules can be film coated by known methods.

The granules, prepared according to present invention are used in the manufacture of tablets, but, they may also suitable for the preparation of pharmaceutical dosage forms such as hard gelatin capsule or sachets or instant granules.

Preferred pharmaceutical oral dosage form is tablet which can be optionally coated by methods well known in the art.

Examples to pharmaceutically acceptable film forming agents or ready-to-use mixtures, without limiting the scope of the invention, are hydroxypropylmethyl cellulose, polyvinyl alcohol. Film coating solution can also contain further functional excipients such as plasticizers, opacifiers and colouring agents.

All granules formulated are successfully compressed resulting in uniform tablets. The tablets were tested in relation to weight uniformity, content uniformity, hardness, friability, disintegration time and dissolution.

### Example Intragranular Part of imatinib tablet

**Table 3**

| **Intragranular Ingredients** | **(mg/tablet)** | **%w/w** |
|---|---|---|
| imatinib mesylate | 119.5 | 8.33 |
| Microcrystalline cellulose (PH 101) | 230.0 | 16.03 |
| HPMC E3 | 35.0 | 2.44 |
| Crospovidone | 129.75 | 9.04 |
| Total Inner Phase | 514.25 | 35.82 |

Extragranular Part of high load imatinib tablet

**Table 4**

| **Extragranular Ingredients** | **(mg/tablet)** | **% w/w** |
|---|---|---|
| imatinib mesylate | 358.5 | 25.00 |
| Microcrystalline cellulose (PH 102) | 373.0 | 26.00 |
| Crospovidone | 129.75 | 9.04 |
| Colloidal Silicon Dioxide | 11.0 | 0.76 |
| Mg Stearate | 13.50 | 0.94 |
| **Total Extragranular Phase** | **885.75** | **61.74** |

Film Coating

**Table 5**

| **Film Coating** | **(mg/tablet)** | **% w/w** |
|---|---|---|
| Opadry II Orange | 35.0 | 2.44 |
| Water | 315.0 | 21.95 |
| Total Film Coating | 350.00 | 24.2 |

### Film Coated Tablet Manufacturing Process

There is provided a process for the film coated tablet preparation of a pharmaceutical form of imatinib, suitable for oral administration, said process comprising following steps:

**a. Intragranulation**

1. The ¼ part of imatinib mesylate, microcrystalline cellulose pH101, the 1/2 part of crospovidone, hypromellose E3 are weighted, sieved from a screen of 16 mesh.

2. Mixture prepared at step (1) is mixed for 10 min. in high shear mixer granulator dryer.

3. Purified water is added onto the mixture in the granulator to form granules.

4.The blend is mixed and dried in the same equipment until proper moisture then sizing the dried granules by using suitable milling equipment

5. The obtained granules are sieved through 16 mesh sieve.

**b. Extragranulation**

6. The rest of imatinib mesylate, and crospovidone and microcrystalline pH 102, colloidal silicon dioxide are weighted and sieved from a screen of 16 mesh.

7. Mixture prepared in step (6) and granules obtained in step (5) are transferred to the high shear mixer granulator dryer and mixed for 15 min.

8. Mg stearate is sieved from a screen of 16 mesh and added to the mixture prepared in step (7) and mixed.

9. The final blend is compressed on a tabletting machine using appropriate punches.

10. The compressed tablets are compacted in a compactor and retabletted to reach 1400 mg of total tablet weight.

11. Coating agent or mixtures of coating agents are added into purified water and stirred.

12. Core tablets are installed into a coating pan and coated with the coating suspension prepared at step (10).

## Claims

1. A pharmaceutical composition comprising an intra-granular and an extra-granular fraction of imatinib or pharmaceutical salts thereof **characterized in that** a substantial part of imatinib is added extragranularly and separated from intragranularly added imatinib or pharmaceutical salts thereof.

2. A pharmaceutical composition as claim 1, wherein the ratio of the intragranular fraction to extragranular fraction of imatinib or pharmaceutical salts thereof is in the range of 1:10 to 1:1.

3. A pharmaceutical composition as claim 1, the ratio of the intragranular fraction to extragranular fraction of imatinib or pharmaceutical salts thereof is in the range of 1:3.

4. According to claim 1, a wet granulation method of imatinib or pharmaceutically acceptable salt thereof characterized granulation is performed in low shear and/or high shear and/or fluid bed granulation.

5. According to claim 1, the wet granulation, as claimed in claim 1, comprises following stepsa)charging a part of imatinib drug substance, microcrystalline cellulose, a part of crospovidone, hydroxypropyl methylcellulose in mixer-granulator; (b) blending mixture;(c) granulating the material using purified water; (d) All the powders(drug and excipients)were brought to a standard particle size by passing through an appropriate size screen; (e) drying the milled granulation; (f) milling the dried granulation through an appropriate size screen; (g) charging dried granulation along with microcrystalline cellulose, croscarmellose sodium, and colloidal silicon dioxide into a diffusion blender and blending the material for an appropriate amount of time; (h) charging the magnesium stearate into the diffusion blender and blending for an appropriate amount of time; (i) compressing the blended powders on a high-speed rotary press; and (j) film coating the tablets.

6. A pharmaceutical composition as claim 1, comprising imatinib or pharmaceutically acceptable salt granules or powder mixture with compressibility index <20, preferably 12.50.
